# EUROPÄISCHE PATENTSCHRIFT

(11) **EP 0 648 724 B1**
(45) Veröffentlichungstag und Bekanntmachung des Hinweises auf die Patenterteilung: **30.07.1997**
(21) Anmeldenummer: 94114937.9
(22) Anmeldetag: 22.09.1994
(51) Int. Cl.: C07C 17/00, C07C 25/13

(54) **Verfahren zur Herstellung von 1-Brom-3,5-difluorbenzol**
Process for the preparation of 1-Bromo-3,5-difluorobenzene
Procédé pour la préparation de 1-Bromo-3,5-difluorobenzène

(30) Priorität: 08.10.1993 DE 4334437
(43) Veröffentlichungstag der Anmeldung: 19.04.1995
(73) Patentinhaber: HOECHST AKTIENGESELLSCHAFT, 65929 Frankfurt am Main (DE)
(72) Erfinder: Neuber, Marita, Dr., D-60528 Frankfurt am Main (DE); Leupold, Ernst Ingo, Dr., D-61267 Neu Anspach (DE); Litterer, Heinz, Dr., D-65307 Bad Schwalbach (DE); Bartels, Günter, Dr., D-30938 Burgwedel (DE); Kanschik-Conradsen, Andreas, Dr., D-30826 Garbsen (DE)

(56) Entgegenhaltungen:
- EP-A- 0 046 665
- DATABASE WPI Section Ch, Week 9232, Derwent Publications Ltd., London, GB; Class E14, AN 92-265123 & JP-A-4 182 440 (ISHIHARA SANGYO KAISHA LTD) 30. Juni 1992
- PATENT ABSTRACTS OF JAPAN vol. 007, no. 259 (C-195) 18. November 1983 & JP-A-58 144 330 (TORAY KK) 27. August 1983

## Beschreibung

Die vorliegende Erfindung betrifft ein Verfahren zur Herstellung von 1-Brom-3,5-difluorbenzol.

1-Brom-3,5-difluorbenzol ist ein Vorprodukt für die Herstellung von Pharmazeutika.

Nach dem Stand der Technik kann 1-Brom-3,5-difluorbenzol durch folgende Verfahren hergestellt werden:
1. durch Bromierung von 2,4-Difluoranilin, anschließende Diazotierung mit NaNO₂ und Deaminierung durch unterphosphorige Säure in saurer Lösung.
   A. Roe und W. F. Little, J. Org. Chem. 20 (1955) 1577/1590 erhalten nach diesem Verfahren 1-Brom-3,5-difluorbenzol in einer Ausbeute von 60 %.
   L. I. Kruse et al., J. Org. Med., 29 (1986) 887/889 und J. Org. Med., 30 (1987) 486/494 erzielen eine Gesamtausbeute an 1-Brom-3,5-difluorbenzol von 57 %
   In US 5 157 169 ist eine Ausbeute an 1-Brom-3,5-difluorbenzol von 70 % beschrieben.
   Die nach diesem Verfahren erzielten Ausbeuten sind gering, teure Ausgangsmaterialien gehen verloren. Das Verfahren ist vielstufig und insbesondere bei den Verfahrensschritten Diazotierung und Deaminierung fallen als Koppelprodukte Säuren und Salze an. Für die Herstellung von 1-Brom-3,5-difluorbenzol in einem größeren Maßstab ist dieses Verfahren daher wenig geeignet.
2. durch photochemische Bromierung von m-Difluorbenzol
   R. Bolton und E. S. E. Owen, J. Fluor. Chem. 46 (1990) 393/406 beschreiben, daß nach diesem Verfahren 19 % 1-Brom-3,5-difluorbenzol neben 10 % der anderen Bromdifluorbenzol-Isomeren und 43 % an höherbromierten Difluorbenzolen entstehen. Bei diesem Verfahren ist die Bildung von 1-Brom-3,5-difluorbenzol wenig selektiv.
   Durch direkte Bromierung von m-Difluorbenzol ist 1-Brom-3,5-difluorbenzol nicht erhältlich. Dabei entsteht selektiv 1-Brom-2,4-difluorbenzol.
3. durch Isomerisierung von 1-Brom-2,4-difluorbenzol
   In EP 63066 wird die Isomerisierung von 1-Brom-2,4-difluorbenzol in Gegenwart von Alkalibasen, bevorzugt Alkaliamiden, und macrocyclischen Verbindungen wie z. B. Polyethern durchgeführt.
   Dieses Verfahren weist eine Reihe von Nachteilen auf. Die makrocyclischen Verbindugen haben eine komplizierte Struktur und sind daher sehr teuer. Sie werden nach der Reaktion nicht zurückgewonnen. Zur Produktaufarbeitung muß das basische Reaktionsgemisch neutralisiert werden, wobei Salze anfallen. Wegen der geringen Ausbeuten an 1-Brom-3,5-difluorbenzol von 63 % und der hohen Materialkosten ist dieses Verfahren wenig wirtschaftlich.
   In JP 04 182 440 (CA 117: 170951n) wird die Isomerisierung von 1-Brom-2,4-difluorbenzol in Gegenwart von Alkalimetall-t-butoxiden oder Aluminiumhalogeniden beschrieben. Auch bei diesem Verfahren ist ein zusätzlicher, salzbildender Neutralisationsschritt erforderlich. Die Ausbeute an 1-Brom-3,5-difluorbenzol beträgt nur 41,4 %.

Andere Einsatzmaterialien sind für die Herstellung von 1-Brom-3,5-difluorbenzol in der Literatur nicht beschrieben.

Es bestand somit ein Bedarf nach einem Verfahren, das die beschriebenen Nachteile vermeidet und 1-Brom-3,5-difluorbenzol technisch einfach zugänglich macht, Nebenprodukte vermeidet und zu einer geringen Umweltbelastung führt.

Diese Aufgabe wird gelöst durch ein Verfahren zur Herstellung von 1-Brom-3,5-difluorbenzol, dadurch gekennzeichnet, daß 1-Brom-2,4-difluorbenzol oder ein Gemisch aus 1-Brom-2,4-difluorbenzol und 1-Brom-2,6-difluorbenzol an sauren Zeolithen vom Pentasil-Typ isomerisiert wird.

In JP 58-144 330 wird die Isomerisierung von Di- und Trichlorbenzol an sauren Zeolithen beschrieben. Das Verfahren soll generell auf polyhalogenierte monocyclische Aromaten anwendbar sein, wobei als Halogene Fluor, Chlor, Brom und Jod beansprucht werden. Als erfindungsgemäße Zeolithe sollen alle sauren Zeolithe geeignet sein, in deren Poren die Edukte und Produkte diffundieren können.

Es wurde nun im Gegensatz zur Lehre der JP 58-144 330 überraschenderweise gefunden, daß saure Zeolithe nicht generell geeignet sind, die Isomerisierung von polyhalogenierten monozyklischen aromatischen Verbindungen zu katalysieren. Während aus JP 58-144 330 hervorgeht, daß auch Zeolithe von Mordenit-Typ geeignet sein sollen, eine Isomerisierung polyhalogenierter monozyklischer Aromaten herbeizuführen, dies allerdings nur für Di- und Trichlorbenzole belegt wird, zeigt es sich hingegen, daß für die Isomerisierung von 1-Brom-2,4-difluorbenzol Zeolithe vom Mordenit-Typ in keiner Weise geeignet sind. Saure Mordenite führen bevorzugt zu unerwünschten Nebenreaktionen wie Abspaltung von HBr und Transbromierung. So führt die Verwendung eines sauren Mordenits schon bei einem Umsatz von 10% mit einer Selektivität von 75% zu m-Difluorbenzol und Difluordibrombenzolen.

Die Lehre von JP 58-144 330 gilt weiterhin nicht für alle halogenierten Aromaten. Da ein Fluoratom am aromatischen Ring nicht beweglich ist, sind ausschließlich mit Fluor substituierte Verbindungen wie m-Difluorbenzol nicht isomerisierbar (J. Pardillos et al., J. Am. Chem. Soc., 112 (1990) 1313/1318).

Vor diesem Hintergrund war es nicht zu erwarten, daß ein saurer Zeolith überhaupt für die Isomerisierung von 1-Brom-2,4-difluorbenzol zu 1-Brom-3,5-difluorbenzol in Betracht kommen würde. Umso überraschender ist es, daß Zeolithe vom Pentasil-Typ sich für diese Art der Isomerisierung sehr gut eignen. Insbesondere ist überraschend, daß sich 1-Brom-2,6-difluorbenzol zu 1-Brom-3,5-difluorbenzol isomerisieren läßt, da der intramolekularer Mechanismus, wie er für die Isomerisierung von halogenierten Aromaten beschrieben ist (J. Pardillos et al., J. Am. Chem. Soc., 112 (1990) 1313/1318), nicht ablaufen kann. Mit sauren Mordeniten zeigt sich erwartungsgemäß, daß von 1-Brom-2,6-difluorbenzol Brom entweder als HBr abgespalten oder auf ein anderes Bromdifluorbenzolmolekül übertragen wird. So beträgt die Selektivität zu m-Difluorbenzol und Dibromdifluorbenzolen 93% bei einem Umsatz von 6%, wenn ein Gemisch aus 1-Brom-2,4-difluorbenzol und 1-Brom-2,6-difluorbenzol zur Reaktion eingesetzt wird.

Zeolithe vom Pentasil-Typ, die sich als Katalysatoren für das erfindungsgemäße Verfahren einsetzen lassen, gehören zu den mittelporigen Zeolithen, deren Porenöffnungen durch 10 Tetraederatome gebildet werden.
Als Tetraederatome werden Al- und Si-Atome bezeichnet, die tetraedrisch von Sauerstoffatomen umgeben sind. Diese Tetraeder sind über gemeinsame Sauerstoffatome verknüpft und bilden eine Kristallstruktur, die von definierten Poren und Hohlräumen durchzogen ist. Charakteristisch für die Pentasile ist eine Struktur, die aus den sogenannten Pentasil-Einheiten aufgebaut werden kann. Die beiden Endglieder aller Pentasil-Strukturen sind ZSM-5 (MFI-Struktur) und ZSM-11 (MEL-Struktur). Die Kristallstrukturen der Pentasile ist z. B. beschrieben in P. A. Jacobs und J. A. Martens, "Synthesis of High-Silica Zeolites", Elsevier Science Publishers, 1989.

Für die Isomerisierung von Bromdifluorbenzolen hat sich in vielen Fällen der Einsatz von Zeolithen mit der Kristallstruktur von ZSM-5 bewährt.

Es hat sich als günstig erwiesen, wenn das SiO₂/Al₂O₃-Verhältnis der erfindungsgemäßen Zeolithe zwischen 22 und 200, bevorzugt zwischen 24 und 100, liegt.

Die genannten Zeolithe können nach Vorschriften aus der Literatur durch hydrothermale Synthese hergestellt werden. Nach der Kristallisation werden die Zeolithe abfiltriert und getrocknet. Wurde die Synthese in Gegenwart eines organischen Templats durchgeführt, so muß in oxidierender Atmosphäre, bevorzugt an Luft, calciniert werden, um die organische Verbindung aus den Poren zu entfernen. (Die templatfreie Form ist frei von Alkylammonium- oder -phosphoniumionen von Aminen).

Eventuell vorhandene Alkalimetall-Ionen werden anschließend durch Ionenaustausch gegen zwei- oder dreiwertige Metallkationen oder gegen Ammonium-Ionen oder Protonen ausgetauscht. Dabei ist der Ionenaustausch mit NH₄⁺ oder H⁺ ganz besonders bevorzugt. Diese saure Modifizierung ist notwendig, weil der Zeolith sonst keine katalytische Wirkung entfaltet. Dabei ist es zweckmäßig, daß mindestens 50 %, vorzugsweise mindestens 90 % der Alkalimetallionen durch die genannten anderen Ionen ausgetauscht sind. Bei 200 bis 800°C, bevorzugt 400 bis 500°C werden die Zeolithe durch Dehydratisierung (und Deammonisierung bei NH₄⁺-Formen) in katalytisch aktive Form überführt.

Die Zeolithe können für die erfindungsgemäße Verwendung entweder binderfrei verpreßt werden oder mit Hilfe von Bindern in eine geeignete Anwendungsform, z. B. in Strangform gebracht werden. Als Binder sind vor allem Oxide, Hydroxyde oder Hydroxychloride des Aluminiums und die Oxide des Siliziums, Titans und Zirkons sowie Tonmaterialien geeignet.

Die Temperatur für die Isomerisierung kann zwischen ca. 250 und 550°C liegen, bevorzugt zwischen 275 und 450°C.

Das erfindungsgemäße Verfahren kann sowohl diskontinuierlich als auch kontinuierlich durchgeführt werden.

Die diskontinuierliche Isomerisierung kann am einfachsten in einem Autoklaven durchgeführt werden. Da die Reaktionstemperatur höher als die Siedetemperaturen der Reaktanden und Produkte liegt, wird die Reaktion bei entsprechend erhöhtem Reaktionsdruck durchgeführt.

Der pulverförmige oder zu Pellets verpreßte Katalysator kann im Einsatzgemisch aufgeschlämmt werden. Das Massenverhältnis von Einsatzgemisch zu Katalysator kann im Bereich von 1:1 bis 1000:1, bevorzugt zwischen 5:1 und 50:1 variieren. Es kann in Gegenwart von dem Katalysator gegenüber inerten Lösungsmittel oder ohne Lösungsmittel gearbeitet werden, wobei die Isomerisierung ohne Lösungsmittel bevorzugt ist. Die Reaktionsdauer kann zwischen ca. 1 Stunde und 12 Stunden betragen, je nach Reaktionstemperatur und zu erzielendem Umsatzgrad.

Die kontinuierliche Isomerisierung kann in allen dafür geeigneten Apparaturen wie z. B. in einem Festbett oder einem Wirbelbett durchgeführt werden. Technisch am einfachsten ist ein Festbett-Reaktor zu handhaben. Der Katalysator wird in Form von Pellets in den Reaktor eingefüllt und 1-Brom-2,4-difluorbenzol oder Bromdifluorbenzol-Isomerengemische darübergeleitet.

Die Reaktion kann in Gegenwart von Gasen wie z. B. Stickstoff oder Wasserstoff durchgeführt werden. Die Anwesenheit von Wasserstoff kann die Bildung höhersiedender Produkte wie Dibromdifluorbenzol verringern und die Standzeit des Katalysators verlängern.

Die Katalysatorbehandlung, charakterisiert durch die LHSV (= liquid hourly space velocity, 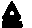 Einsatz pro Stunde pro l Katalysator) kann im Bereich zwischen 0,1 und 5 h⁻¹, bevorzugt zwischen 0,2 und 2 h⁻¹ variiert werden.

Das bei der Reaktion erhaltene Produktgemisch kann durch Destillation getrennt werden. Dabei können HBr und HF, die bei der Reaktion in Spuren entstehen, vor der Destillation durch Waschen mit Wasser oder alkalischen Lösungen entfernt werden oder aber direkt zusammen mit m-Difluorbenzol als Leichtsiederfraktion abgetrennt werden. m-Difluorbenzol kann wieder für die Bromierung eingesetzt werden. Von den 3 isomeren Difluorbenzolen kann 1-Brom-3,5-difluorbenzol als am leichtesten siedendes Isomer abgetrennt werden. Nach der Abtrennung von 1-Brom-3,5-difluorbenzol verbleiben neben 1-Brom-2,4-difluorbenzol auch 1-Brom-2,6-difluorbenzol und höhersiedende Produkte sowie gegebenenfalls geringe Mengen an 1-Brom-3,5-difluorbenzol. Diese Fraktion kann sowohl direkt wieder in die Isomerisierung zurückgeführt werden, als auch durch weitere Destillation von den höhersiedenden Produkten abgetrennt werden. Durch die Rückführung des nicht umgesetzten 1-Brom-2,4-difluorbenzols sowie der bei der Isomerisierung anfallenden Nebenprodukte kann eine hohe Ausbeute an 1-Brom-3,5-difluorbenzol erzielt werden.

Die nachstehenden Beispiele sollen die erfindungsgemäßen Verfahren näher erläutern.

### Beispiele:

Die Beispiele 1 bis 6 sowie V1 und V2 werden im Festbett-Strömungsreaktor bei Atmosphärendruck durchgeführt. 25 ml Katalysator (80 % Zeolithanteil, 20 % Al₂O₃ als Binder, Korngröße 1 bis 2 mm) werden in einen Quarzglasreaktor (20 mm Innendurchmesser), der durch einen elektrischen Ofen beheizt werden kann, gefüllt, 1-Brom-2,4-difluorbenzol wird von oben zudosiert, die Reaktionsprodukte werden am Reaktorausgang kondensiert. Die LHSV beträgt 0,5 h⁻¹. Wenn Trägergas verwendet wird, beträgt die Strömung 3 l/h. Die Zeolithe werden vor der Reaktion in Stickstoff bei 500°C getrocknet.

Die Reaktionsprodukte werden gaschromatographisch analysiert.

Die Ergebnisse der Isomerisierung an verschiedenen Katalysatoren sind in Tabelle 1 zusammengestellt.

**Tabelle 1**

| Bsp. | Zeolith | SiO₂/Al₂O₃ | Trägergas | Tem., °C | Zeit, h | Umsatz mol-% | S_{3,5} mol-% | S_{2,6} mol-% | S_{DFB} mol-% | S_{HS} mol-% |
|---|---|---|---|---|---|---|---|---|---|---|
| 1 | HZSM-5 | 27 | | 300 | 0,3 | 31 | 82 | 9 | 9 | <1 |
| | | | | | 2 | 42 | 79 | 7 | 10 | 4 |
| 2 | HZSM-5 | 27 | | 350 | 0,3 | 66 | 72 | 7 | 17 | 4 |
| | | | | | 2 | 63 | 85 | 8 | 5 | 2 |
| 3 | HZSM-5 | 27 | | 400 | 2 | 48 | 85 | 10 | 5 | 1 |
| 4 | HZSM-5 | 27 | H₂ | 300 | 1,3 | 34 | 74 | 7 | 14 | 4 |
| | | | | 315 | 6,8 | 38 | 88 | 8 | 3 | 1 |
| | | | | 345 | 29,5 | 31 | 89 | 9 | 2 | 0 |
| | | | | 435 | 55,8 | 25 | 84 | 12 | 4 | 0 |
| 5 | HZSM-5 | 30 | H₂ | 300 | 3,3 | 28 | 82 | 6 | 12 | 1 |
| | | | | 335 | 27,5 | 26 | 88 | 6 | 5 | 0 |
| 6 | HZSM-11 | 80 | H₂ | 355 | 5,8 | 16 | 87 | 9 | 3 | 0 |
| | | | | 390 | 14,3 | 22 | 86 | 11 | 3 | 0 |
| V1 | HMordenit | 34 | | 350 | 0,3 | 44 | 15 | 8 | 55 | 22 |
| | | | | | 2,5 | 12 | 14 | 11 | 50 | 24 |
| V2 | HBeta | 24 | | 350 | 0,3 | 27 | 14 | 6 | 53 | 28 |
| | | | | | 2 | 8 | 15 | 8 | 50 | 26 |
| S_{3,5} Selektivität zu 1-Brom-3,5-difluorbenzol S_{2,6} Selektivität zu 1-Brom-2,6-difluorbenzol S_{DFB} Selektivität zu m-Difluorbenzol S_{HS} Selektivität zu Hochsiedern, v.a. Dibromdifluorbenzole | | | | | | | | | | |

### Beispiele 7 und V3:

Die Versuche werden wie in den Beispielen 1 bis 6 beschrieben durchgeführt. Es wird ein Gemisch eingesetzt, das 87 % 1-Brom-2,4-difluorbenzol, 12% 1-Brom-2,6-difluorbenzol und 1 % Dibromdifluorbenzole enthält.
In Tabelle 2 sind die Ergebnisse zusammengestellt.

**Tabelle 2**

| Bsp. | Zeolith | SiO₂/Al₂O₃ | Trägergas | Tem., °C | Zeit, h | Umsatz mol-% | S_{3,5} mol-% | S_{DFB} mol-% | S_{HS} mol-% |
|---|---|---|---|---|---|---|---|---|---|
| 7 | HZSM-5 | 27 | H₂ | 320 | 1,5 | 38 | 86 | 10 | 4 |
| V3 | HMordenit | 34 | H₂ | 320 | 1,5 | 6 | 7 | 58 | 35 |
| Umsatz: Umsatz an 1-Brom-2,4-difluorbenzol und 1-Brom-2,6-difluorbenzol | | | | | | | | | |

### Beispiel 8:

170 g 1-Brom-2,4-difluorbenzol werden in Gegenwart von 5 Gew.-% Katalysator (80 % HZSM-5 (SiO₂/Al₂O₃ = 27), 20 % Al₂O₃ als Binder, Korngröße 1 bis 2 mm, bei 400°C vorgetrocknet) in einem Labor-Rührautoklav 1 Stunde lang auf 320°C erhitzt. Der Druck beträgt ca. 25 bar. Es wird ein Umsatz von 29 % erzielt, die Selektivitäten zu 1-Brom-3,5-difluorbenzol, 1-Brom-2,6-difluorbenzol, m-Difluorbenzol sowie Dibromfluorbenzol betragen 73 mol-%, 7 mol-%, 17 mol-% bzw. 4 mol-%.

Nach Abfiltrieren des Katalysators werden erneut 170 g 1-Brom-2,4-difluorbenzol mit dem gebrauchten Katalysator 1 Stunde lang auf 320°C erhitzt. Der Umsatz sinkt auf 21 %, die Selektivitäten zu 1-Brom-3,5-difluorbenzol, 1-Brom-2,6-difluorbenzol, m-Difluorbenzol sowie Dibromfluorbenzol werden zu 80 mol-%, 8 mol-%, 9 mol-% bzw. 3 mol-% ermittelt.

## Patentansprüche

1. Verfahren zur Herstellung von 1-Brom-3,5-difluorbenzol, dadurch gekennzeichnet, daß 1-Brom-2,4-difluorbenzol oder ein Gemisch aus 1-Brom-2,4-difluorbenzol und 1-Brom-2,6-difluorbenzol an sauren Zeolithen vom Pentasil-Typ isomerisiert wird.

2. Verfahren nach Anspruch 1, dadurch gekennzeichnet, daß der eingesetzte Zeolith die Kristallstruktur von ZSM-5 aufweist.

3. Verfahren nach den Ansprüchen 1 und 2, dadurch gekennzeichnet, daß das SiO₂/Al₂O₃-Verhältnis des eingesetzten Zeoliths zwischen 22 und 200, insbesondere zwischen 24 und 100, liegt.

4. Verfahren nach mindestens einem der Ansprüche 1 bis 3, dadurch gekennzeichnet, daß mindestens 90 % der Kationen aus Protonen oder Ammoniumionen bestehen.

5. Verfahren nach mindestens einem der Ansprüche 1 bis 4, dadurch gekennzeichnet, daß die Reaktionstemperatur zwischen 250 und 500°C, insbesondere 275 und 450°C beträgt.

6. Verfahren nach mindestens einem der Ansprüche 1 bis 5, dadurch gekennzeichnet, daß die Reaktion kontinuierlich im Festbett-Strömungsreaktor durchgeführt wird.

7. Verfahren nach mindestens einem der Ansprüche 1 bis 6, dadurch gekennzeichnet, daß das Verfahren in Gegenwart von Gasen, bevorzugt Wasserstoff durchgeführt wird.

8. Verfahren nach mindestens einem der Ansprüche 1 bis 7, dadurch gekennzeichnet, daß die Katalysatorbelastung 0,1 bis 5, insbesondere 0,2 bis 2 l Einsatz pro l Katalysator pro Stunde beträgt.

9. Verfahren nach mindestens einem der Ansprüche 1 bis 8, dadurch gekennzeichnet, daß der Reaktionsdruck dem Atmosphärendruck entspricht.

10. Verfahren nach mindestens einem der Ansprüche 1 bis 5, dadurch gekennzeichnet, daß bei der diskontinuierlichen Isomerisierung das Massenverhältnis vom Einsatzgemisch zu Katalysator zwischen 1:1 bis 1000:1, insbesondere 5:1 bis 50:1 beträgt.

## Claims

1. A process for preparing 1-bromo-3,5-difluorobenzene, which comprises isomerizing 1-bromo-2,4-difluorobenzene or a mixture of 1-bromo-2,4-difluorobenzene and 1-bromo-2,6-difluorobenzene over acid zeolites of the pentasil type.

2. The process as claimed in claim 1, wherein the zeolite used has the crystal structure of ZSM-5.

3. The process as claimed in claims 1 and 2, wherein the SiO₂/Al₂O₃ ratio of the zeolite used is between 22 and 200, in particular between 24 and 100.

4. The process as claimed in at least one of claims 1 to 3, wherein at least 90% of the cations are protons or ammonium ions.

5. The process as claimed in at least one of claims 1 to 4, wherein the reaction temperature is between 250 and 500°C, in particular between 275 and 450°C.

6. The process as claimed in at least one of claims 1 to 5, wherein the reaction is carried out continuously in a fixed-bed flow reactor.

7. The process as claimed in at least one of claims 1 to 6, wherein the process is carried out in the presence of gases, preferably hydrogen.

8. The process as claimed in at least one of claims 1 to 7, wherein the space velocity is 0.1 to 5, in particular 0.2 to 2, l of feed per l of catalyst per hour.

9. The process as claimed in at least one of claims 1 to 8, wherein the reaction pressure equals atmospheric pressure.

10. The process as claimed in at least one of claims 1 to 5, wherein the feed mixture/catalyst weight ratio in the batchwise isomerization is from 1:1 to 1000:1, in particular from 5:1 to 50:1.

## Revendications

1. Procédé de préparation du 1-bromo-3,5-difluorobenzène, caractérisé en ce qu'on isomérise sur des zéolites acides du type pentasil du 1-bromo-2,4-difluorobenzène ou un mélange de 1-bromo-2,4-difluorobenzène et de 1-bromo-2,6-difluorobenzène.

2. Procédé selon la revendication 1, caractérisé en ce que la zéolite utilisée présente la structure cristalline du ZSM-5.

3. Procédé selon les revendications 1 et 2, caractérisé en ce que le rapport SiO₂/Al₂O₃ de la zéolite utilisée est compris entre 22 et 200, en particulier entre 24 et 100.

4. Procédé selon au moins l'une des revendications 1 à 3, caractérisé en ce qu'au moins 90 % des cations sont constitués de protons ou d'ions ammonium.

5. Procédé selon au moins l'une des revendications 1 à 4, caractérisé en ce que la température de réaction est comprise entre 250 et 500°C, en particulier entre 275 et 450°C.

6. Procédé selon au moins l'une des revendications 1 à 5, caractérisé en ce que la réaction est mise en oeuvre en continu dans un réacteur à écoulement à lit fixe.

7. Procédé selon au moins l'une des revendications 1 à 6, caractérisé en ce que le procédé est mis en oeuvre en présence de gaz, de préférence d'hydrogène.

8. Procédé selon au moins l'une des revendications 1 à 7, caractérisé en ce que la vitesse spatiale horaire du catalyseur est de 0,1 à 5 et en particulier de 0,2 à 2 l par litre de catalyseur par heure.

9. Procédé selon au moins l'une des revendications 1 à 8, caractérisé en ce que la pression de réaction correspond à la pression atmosphérique.

10. Procédé selon au moins l'une des revendications 1 à 5, caractérisé en ce que, lors de l'isomérisation discontinue, le rapport en masse du mélange utilisé au catalyseur est compris entre 1:1 et 1000:1, en particulier entre 5:1 et 50:1.
